# FASCICULE DE BREVET EUROPEEN

(11) **EP 3 374 007 B1**
(45) Date de publication et mention de la délivrance du brevet: **20.07.2022**
(21) Numéro de dépôt: 16798110.9
(22) Date de dépôt: 10.11.2016
(51) Int. Cl.: A61M 5/31

(54) **SERINGUE**
SPRITZE
SYRINGE

(30) Priorité: 12.11.2015 FR 1560823
(43) Date de publication de la demande: 19.09.2018
(73) Titulaire: GUERBET, 93420 Villepinte (FR)
(72) Inventeur: CACLIN, Jérôme, 69360 Saint Symphorien d'Ozon (FR)
(74) Mandataire: Lavoix
(86) Numéro de dépôt international: PCT/EP2016/077307
(87) Numéro de publication internationale: WO 2017/081178

(56) Documents cités:
- CN-A- 102 218 176
- US-A- 4 929 238
- US-A- 5 318 534

## Description

L'invention concerne une seringue.

Les seringues manuelles sont utilisées par le personnel médical pour injecter un fluide sous pression lors d'une procédure médicale, notamment en radiologie interventionnelle. De telles seringues peuvent être utilisées dans le cas de procédures répétitives de remplissage et d'injection, ou dans le cas d'injections à travers un petit cathéter, ou un micro cathéter. De telles seringues comprennent un corps creux contenant la substance à injecter au patient, un piston d'injection et des moyens de préhension prévus sur le corps et sur le piston.

Les seringues connues notamment de US 6616634 et US 8 412 310 ne permettent pas un remplissage à une main de façon pratique et utilisent des moyens de préhension encombrants. En outre, la fin d'injection sur de telles seringues est peu pratique du fait que le pouce du praticien, qui appuie sur le piston, arrive sur un plan proche de celui formé par l'index et le majeur, ce qui induit un contrôle moins efficace de la fin d'injection.

CN-A-10 2 218 176 décrit une seringue comprenant un piston terminé par un organe de préhension. Le piston est formé de plusieurs pièces incluant des ressorts, une tige et une pièce formant une zone élastique présente à l'extrémité du piston.

Lors de l'utilisation de la plupart des seringues de l'art antérieur, les informations relatives aux efforts d'injection fournis par l'utilisateur pour injecter une substance contenue dans ces seringues, ne sont pas transmises suffisamment à l'utilisateur via son contact avec l'organe de préhension de celles-ci. L'utilisateur de la seringue ne peut donc pas finement doser son effort en fonction des informations qu'il reçoit au cours de l'injection.

C'est à ces inconvénients qu'entend plus particulièrement remédier l'invention en proposant une nouvelle seringue, dont la manipulation est plus pratique.

A cet effet, l'invention concerne une seringue comprenant :
- un corps creux allongé formant un réservoir destiné à contenir une substance à injecter, le corps comprenant un embout destiné à recevoir un dispositif de connexion et une ouverture située du côté opposé du corps par rapport à l'embout,
- un piston monté coulissant dans le corps et mobile en translation selon un axe longitudinal de la seringue entre une position rentrée, dans laquelle le piston est en butée du côté de l'embout, et une position sortie, dans laquelle le piston est en butée du côté de l'ouverture,
- des organes de préhension prévus sur le corps, et un organe de préhension prévu à une extrémité du piston opposée par rapport à une tête du piston.

Cette seringue est caractérisée en ce que les organes de préhension prévus sur le corps comprennent des zones d'appui pour des doigts d'un utilisateur, orientées en direction de l'organe de préhension du piston, en ce que l'organe de préhension du piston comprend au moins une zone d'appui pour des doigts de l'utilisateur, située en regard des organes de préhension prévus sur le corps en ce que le piston est formé d'une seule pièce en un matériau ayant un module d'Young supérieur à 3000 MPa, et en ce que le piston est dépourvu de zone élastique entre son organe de préhension et la substance à injecter.

Grâce à l'invention, la seringue peut être facilement utilisée à une main pour les injections et les remplissages, et le praticien peut facilement adapter sa prise de la seringue grâce à la possibilité de rotation des organes de préhension prévus sur le corps et de l'appuie-doigt du piston.

Selon des aspects avantageux mais non obligatoires de l'invention, une telle seringue peut incorporer une ou plusieurs des caractéristiques suivantes, prises dans toute combinaison techniquement admissible :
- Le piston est mobile en rotation par rapport au corps autour de l'axe longitudinal de la seringue.
- L'organe de préhension du piston comprend une zone d'appui située à l'opposé de la tête du piston.
- L'embout du corps comprend une collerette périphérique dont une surface externe est pourvue de nervures de préhension s'étendant parallèlement à l'axe longitudinal de la seringue.
- La zone d'appui de l'organe de préhension du piston située en regard des organes de préhension prévus sur le corps comprend des formes concaves prévue de part et d'autre de la tige du piston.
- Dans la position rentrée du piston, l'organe de préhension du piston est éloigné des organes de préhension prévus sur le corps d'une distance au moins égale à 15 mm.
- Les zones d'appui des organes de préhension prévus sur le corps comprennent des formes concaves prévues de part et d'autre de l'ouverture du corps creux et faisant face à la zone d'appui du piston, ces formes concaves étant ménagées sur deux pattes radiales formant chacune l'un des organes de préhension prévus sur le corps.
- Les organes de préhension prévus sur le corps sont ménagés sur une seule pièce, cette pièce étant montée rotative sur le corps autour de l'axe longitudinal de la seringue.
- Les deux zones d'appui concaves sont ménagées sur la pièce sur laquelle sont ménagés les organes de préhension prévus sur le corps, sur les parties qui relient ces organes de préhension.
- Les deux zones d'appui concaves sont orientées en direction de l'organe de préhension, permettant l'appui d'un doigt de l'utilisateur selon une direction radiale par rapport à l'axe longitudinal de la seringue.
- L'organe de préhension du piston a une forme ovale.

L'invention sera mieux comprise et d'autres avantages de celle-ci apparaitront plus clairement à la lumière de la description qui va suivre d'une seringue conforme à son principe, faite à titre d'exemple non limitatif en référence aux dessins annexés dans lesquels :
- la figure 1 est une vue en perspective d'une seringue conforme à l'invention,
- la figure 2 est une vue en perspective, sous un autre angle, de la seringue de la figure 1,
- la figure 3 est une vue latérale de la seringue des figures 1 et 2,
- la figure 4 est une vue en perspective d'un piston de la seringue des figures 1 à 3,
- la figure 5 est une vue latérale du piston de la figure 4, sur laquelle des positions de doigts sont représentées par des cercles en traits discontinus,
- la figure 6 est une vue latérale d'un organe de préhension de la seringue de la figure 1, sur laquelle des positions de doigts sont représentées par des cercles en traits discontinus,
- la figure 7 est une vue latérale d'une partie de la seringue de la figure 1, dans une seconde configuration, et sur laquelle les positions de doigts sont représentées par des cercles en traits discontinus,
- la figure 8 est une vue similaire à la figure 7, sur laquelle des positions de doigts différentes sont représentées par des cercles en traits discontinus.

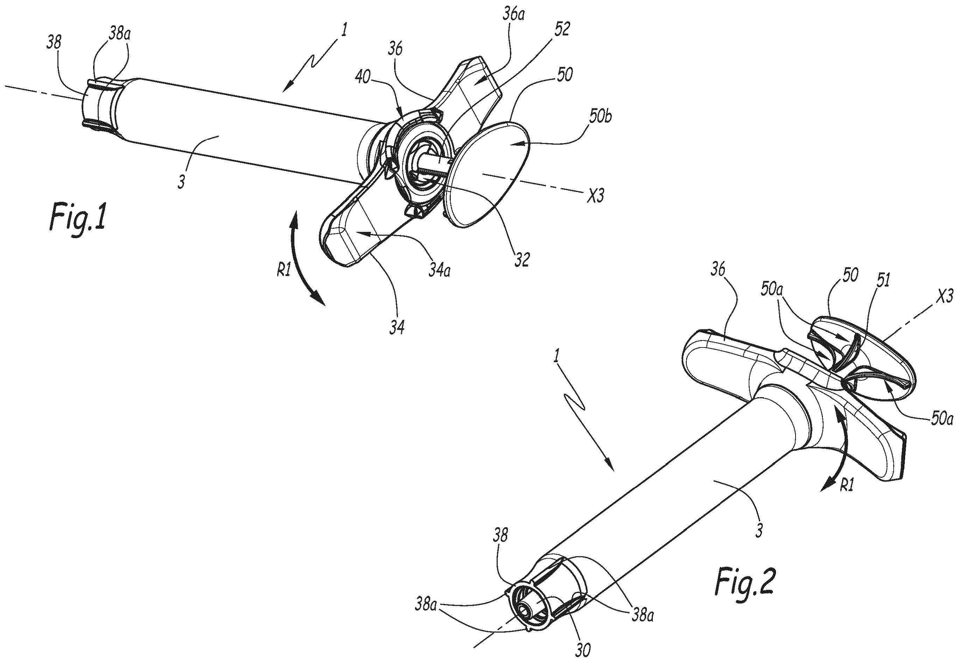

La figure 1 représente une seringue 1 adaptée pour une manipulation manuelle. La seringue 1 comprend un corps creux allongé 3 centré sur un axe longitudinal X3 qui forme également un axe longitudinal de la seringue 1. Le corps 3 forme un réservoir destiné à contenir des substances à injecter. Le corps 3 comprend un embout 30 destiné à recevoir un dispositif de connexion. Cet embout 30 peut être avantageusement un cône LUER, la connexion Luer répondant à des normes telles que les normes ISO 594 et ISO 80369, sans que l'indication de ces normes ne soit limitative. Du côté opposé du corps 3 par rapport à l'embout 30, le corps 3 comprend une ouverture 32.

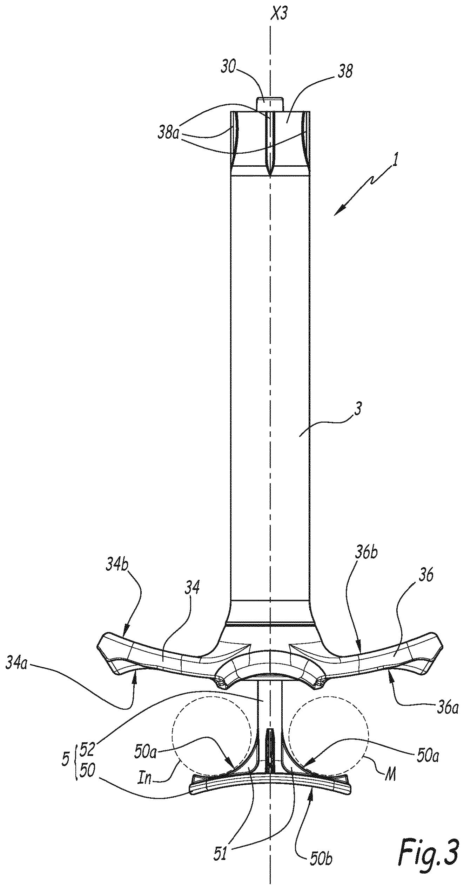

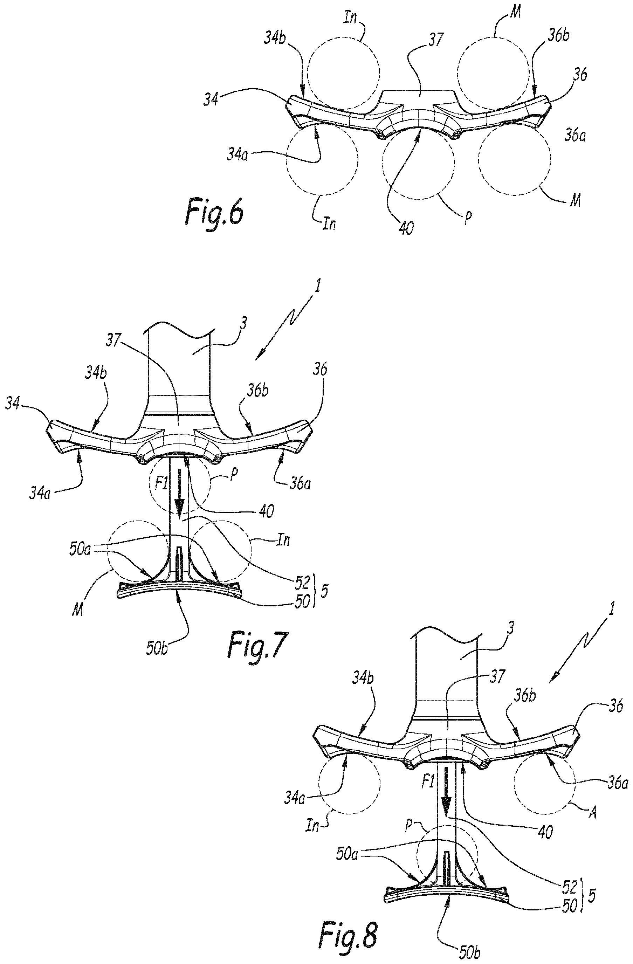

La seringue 1 comprend également un piston 5 monté dans le corps 3 de façon mobile en translation selon l'axe X3 entre une position rentrée, représentée sur les figures 1 et 3, dans laquelle le piston 5 est en butée du côté de l'embout 30, et une position sortie, dans laquelle le piston 5 est en butée du côté de l'ouverture 32. Le piston 5 comprend une tige 52 qui sort du corps 3 par l'ouverture 32 de la seringue 1. Le piston 5 comprend à l'une de ses extrémités, une tête 57 adaptée pour être en contact avec une substance à injecter présente dans le corps 3, et à l'autre de ses extrémités, un organe de préhension 50. La seringue 1 est représentée sur les figures 7 et 8 dans une position intermédiaire, dans laquelle le piston 5 est partiellement sorti du corps 3. Le piston 5 est mobile en rotation par rapport au corps 3 autour de l'axe longitudinal X3 de la seringue.

La seringue 1 comprend des organes de préhension permettant à un praticien de manipuler la seringue pour la remplir d'une substance, et pour administrer la substance à un patient. Le corps 3 comprend à cet effet des organes de préhension 34 et 36 prévus autour de l'ouverture 32, et formés chacun par une patte s'étendant radialement par rapport à l'axe X3. L'organe de préhension 50 prévu à l'extrémité du piston 5 opposée par rapport à la tête 57 est compris dans les organes de préhension de la seringue 1. L'organe de préhension 50 est formé par une plaque de forme ovale s'étendant globalement radialement par rapport à la tige 52.

De manière préférentielle, les organes de préhension 34 et 36 prévus sur le corps 3 sont mobiles en rotation par rapport au corps 3 autour de l'axe X3 de la seringue 1.

Conformément à l'invention, les organes de préhension 34 et 36 prévus sur le corps 3 comprennent des zones d'appui 34a et 36a pour des doigts d'un utilisateur, ces zones d'appui étant orientées en direction de l'organe de préhension 50 du piston 5, et l'organe de préhension 50 du piston 5 comprend au moins une zone d'appui 50a pour des doigts de l'utilisateur, située en regard des organes de préhension 34 et 36 du corps 3. Les zones d'appui 34a et 36a comprennent des formes concaves prévues de part et d'autre de l'ouverture 32 du corps 3 sur chacun des organes de préhension 34 et 36.

Cette combinaison de caractéristiques permet le placement des doigts de l'utilisateur entre les organes de préhension 34 et 36 et l'organe de préhension 50 de manière à autoriser la manipulation de la seringue 1 à une seule main lors de son remplissage, c'est-à-dire lorsque le piston 5 est progressivement sorti du corps allongé 3 selon la flèche F1 de manière à aspirer une substance dans le réservoir de la seringue 1. La possibilité de rotation, représentée par les doubles flèches R1, des organes de préhension 34 et 36 par rapport au corps 3 permet à l'utilisateur de bénéficier d'un positionnement plus confortable de sa main et de ses doigts en fonction de sa position par rapport au patient et de manipuler la seringue 1 avec plus de facilité et de sécurité.

Les organes de préhension 34 et 36 sont de préférence ménagés sur une seule pièce 37 montée rotative sur le corps 3 autour de l'axe X3. La pièce 37 est de préférence fabriquée indépendamment du corps 3. De manière préférentielle, la pièce 37 est montée sur le corps 3 du côté de l'embout 30 jusqu'à l'ouverture 32 et fixée sur cette ouverture 32 par clipsage. De manière préférentielle, la pièce 37 est fixée sur le corps 3 par déformation élastique de cette pièce.

La zone d'appui 50a comprend des formes concaves prévues de part et d'autre de la tige 52. Ces formes concaves sont de préférence ménagées sur des nervures 51 qui relient la tige 52 à l'organe de préhension 50. Le piston 5 comprend de préférence quatre nervures 51 formant les zones d'appui 50a, orientées à 90° les unes par rapport aux autres autour de la tige 52.

Le corps 3 comprend également deux zones d'appui concaves 40 prévues sur le pourtour de l'ouverture 32 et situées entre les organes de préhension 34 et 36. Ces zones d'appui concaves 40 sont orientées en direction de l'organe de préhension 50, et permettent l'appui d'un doigt de l'utilisateur selon une direction radiale par rapport à l'axe X3. Les zones d'appui 40 sont de préférence ménagées sur la pièce 37 sur les parties qui relient les organes de préhension 34 et 36. Les zones d'appui 40 sont avantageusement formées par des bourrelets arrondis centrés sur l'axe X3.

Ainsi, lors du remplissage de la seringue 1, un pouce P de l'utilisateur peut être appuyé contre l'une des zones d'appui 40, tandis qu'un majeur M et un index In de l'utilisateur sont appuyés sur les zones d'appui 50a du piston 5. Ce positionnement de doigts est représenté à la figure 7.

En variante, lors du remplissage de la seringue 1, le pouce P de l'utilisateur peut être appuyé sur la zone d'appui 50a, tandis que un annulaire A de l'utilisateur et l'index In sont respectivement appuyés sur les zones d'appui 34a et 36a. Ce positionnement de doigts est représenté à la figure 8.

Les organes de préhension 34 et 36 comprennent également des zones d'appui 34b et 36b situées à l'opposé des zones d'appui 34a et 36a, c'est-à-dire orientées en direction de l'embout 30 du corps 3. Les zones d'appui 34b et 36b sont utilisées pour placer l'index In et le majeur M lors de l'injection d'une substance.

Dans la première position rentrée du piston 5, l'organe de préhension 50 est éloigné des organes de préhension 34 et 36 d'une distance au moins égale à 15 mm, de préférence au moins égale à 20 mm. Cela permet que, lors de la fin de l'injection d'un produit à un patient, les doigts de l'utilisateur appuyés sur les organes de préhension 34 et 36, et le pouce de l'utilisateur appuyé sur le piston 5 ne soient pas trop rapprochés, afin que l'utilisateur puisse mieux maitriser son geste et sa prise sur la seringue 1. Cela permet également le placement des doigts de l'utilisateur, par exemple l'index In et le majeur M, entre le piston 5 et le corps 3 lorsque la seringue 1 doit être remplie à nouveau, comme montré sur la figure 3.

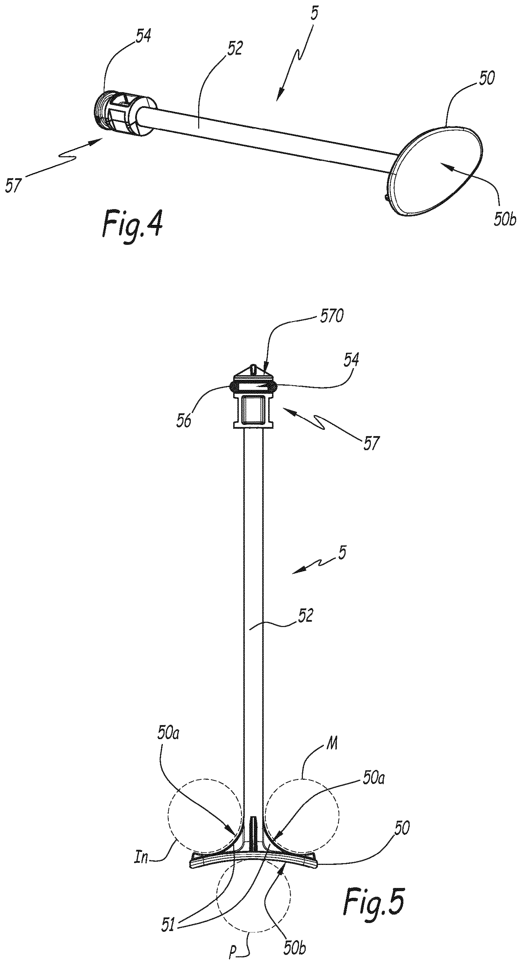

De manière préférentielle, la tête 57 du piston 5 comprend une gorge périphérique 54 et un joint torique 56 monté dans la gorge 54 et s'étendant entre le piston 5 et une paroi interne du corps 3 de manière à assurer l'étanchéité de la seringue 1. Ce mode de réalisation est illustré à la figure 5.

De manière préférentielle, le piston 5 est dépourvu de zone élastique entre l'organe de préhension 50 et la substance à injecter, avec laquelle la tête 57 du piston 5 est en contact.

Le piston 5 est de préférence conçu en une seule pièce très rigide, de préférence en un matériau ayant un module d'Young supérieur à 3000 MPa, de manière plus préférentielle supérieur à 7000 MPa, de manière encore plus préférentielle supérieur à 11000 MPa. Grâce à la rigidité du piston 5 et à la conception de celui-ci en une seule pièce, les informations relatives aux efforts d'injection, que l'on qualifie préférentiellement d'informations tactiles, sont transmises directement à la main de l'utilisateur, ce qui permet à celui-ci de maitriser la manipulation de la seringue et de mieux maitriser l'injection. En outre, l'utilisation du joint torique 56 sur la tête 57 du piston 5 permet de s'affranchir de l'utilisation d'un corps plastique d'étanchéité en bout du piston 5, qui est en contact avec la substance à injecter, comme cela est réalisé généralement dans l'art antérieur. Un corps plastique d'étanchéité et/ou l'utilisation d'un piston composé de plusieurs pièces et/ou d'un piston réalisé dans un matériau moins rigide présente l'inconvénient majeur de créer une ou plusieurs zones, en particulier des zones élastiques, entre la substance à injecter et le pouce de l'utilisateur, le long de l'axe longitudinal X3, dans lesquelles une partie des informations tactiles relatives aux efforts d'injection se perd.

De manière encore plus préférentielle, le piston 5 est conçu en une seule pièce en un matériau ayant un module d'Young supérieur à 3000 MPa et ce piston 5 est dépourvu de zone élastique entre son organe de préhension 50 et la substance à injecter. En d'autres termes, le piston 5 ne comprend pas de parties aptes à se déformer de façon importante selon l'axe longitudinal X3 entre l'organe de préhension 50 et une surface 570 de la tête 57 qui est adaptée pour être en contact avec la substance à injecter. Ainsi, dans ces modes préférentiels de réalisation, les informations tactiles relatives aux efforts d'injection sont préservées et sont ainsi directement ressenties par l'utilisateur.

L'organe de préhension 50 comprend une zone d'appui 50b située à l'opposé de la tête 57 du piston 5. Dans l'exemple représenté, cette zone d'appui 50b est concave, ce qui permet, par exemple, l'appui du pouce P. Selon un mode de réalisation non représenté, cette zone d'appui 50b peut être convexe, ce qui permet l'utilisation de la paume de la main pour pousser le piston 5 dans le corps 3.

L'embout 30 est entouré d'une collerette périphérique 38 dont une surface externe est pourvue de nervures de préhension 38a, s'étendant parallèlement à l'axe X3. Ces nervures de préhension permettent la rotation de la seringue 1 à l'aide des doigts, par exemple lorsqu'un connecteur LUER doit être adapté sur l'embout 30.

## Revendications

1. Seringue (1) comprenant :
- un corps (3) creux allongé formant un réservoir destiné à contenir une substance à injecter, le corps (3) comprenant un embout (30) destiné à recevoir un dispositif de connexion et une ouverture (32) située du côté opposé du corps (3) par rapport à l'embout (30),
- un piston (5) monté coulissant dans le corps (3) et mobile en translation selon un axe longitudinal (X3) de la seringue entre une position rentrée, dans laquelle le piston (5) est en butée du côté de l'embout (30), et une position sortie, dans laquelle le piston (5) est en butée du côté de l'ouverture (32),
- des organes de préhension (34, 36) prévus sur le corps (3), et un organe de préhension (50) prévu à une extrémité du piston (5) opposée par rapport à une tête (57) du piston (5),
les organes de préhension (34, 36) prévus sur le corps (3) comprenant des zones d'appui (34a, 36a) pour des doigts (In, M, P) d'un utilisateur, orientées en direction de l'organe de préhension (50) du piston (5), l'organe de préhension (50) du piston (5) comprenant au moins une zone d'appui (50a) pour des doigts (In, M, P) de l'utilisateur, située en regard des organes de préhension (34, 36) prévus sur le corps (3),
**caractérisée en ce que** le piston (5) est formé d'une seule pièce en un matériau ayant un module d'Young supérieur à 3000 MPa et **en ce que** le piston (5) est dépourvu de zone élastique entre son organe de préhension (50) et la substance à injecter.

2. Seringue selon la revendication 1, **caractérisée en ce que** le piston (5) est mobile en rotation par rapport au corps (3) autour de l'axe longitudinal (X3) de la seringue (1).

3. Seringue selon l'une des revendications précédentes, **caractérisée en ce que** l'organe de préhension (50) du piston (5) comprend une zone d'appui (50b) située à l'opposé de la tête (57) du piston (5).

4. Seringue selon l'une des revendications précédentes, **caractérisée en ce que** l'embout (30) du corps (3) comprend une collerette périphérique (38) dont une surface externe est pourvue de nervures de préhension (38a) s'étendant parallèlement à l'axe longitudinal (X3) de la seringue (1).

5. Seringue selon l'une des revendications précédentes, **caractérisée en ce que** la zone d'appui (50a) de l'organe de préhension (50) du piston (5) située en regard des organes de préhension (34, 36) prévus sur le corps (3) comprend des formes concaves prévue de part et d'autre de la tige (52) du piston (5).

6. Seringue selon l'une des revendications précédentes, **caractérisée en ce que** dans la position rentrée du piston (5), l'organe de préhension (50) du piston (5) est éloigné des organes de préhension (34, 36) prévus sur le corps (3) d'une distance au moins égale à 15 mm.

7. Seringue selon l'une des revendications précédentes, **caractérisée en ce que** les zones d'appui (34a, 36a) des organes de préhension (34, 36) prévus sur le corps (3) comprennent des formes concaves prévues de part et d'autre de l'ouverture (32) du corps creux et faisant face à la zone d'appui du piston (5), ces formes concaves étant ménagées sur deux pattes radiales formant chacune l'un des organes de préhension (34, 36) prévus sur le corps (3).

8. Seringue selon l'une des revendications précédentes, **caractérisée en ce que** les organes de préhension (34, 36) prévus sur le corps (3) sont ménagés sur une seule pièce (37), cette pièce (37) étant montée rotative sur le corps (3) autour de l'axe longitudinal (X3) de la seringue.

9. Seringue selon la revendication 8, **caractérisée en ce que** deux zones d'appui concaves (40) sont ménagées sur la pièce (37) sur laquelle sont ménagés les organes de préhension (34,36) prévus sur le corps (3), sur les parties qui relient ces organes de préhension (34, 36).

10. Seringue selon la revendication précédente, **caractérisée en ce que** les deux zones d'appui concaves (40) sont orientées en direction de l'organe de préhension (50), permettant l'appui d'un doigt de l'utilisateur selon une direction radiale par rapport à l'axe longitudinal (X3) de la seringue.

11. Seringue selon l'une des revendications précédentes, **caractérisée en ce que** l'organe de préhension (50) du piston (5) a une forme ovale.

## Patentansprüche

1. Spritze (1), umfassend:
- einen länglichen hohlen Körper (3), der einen Behälter bildet, der dazu bestimmt ist, eine zu injizierende Substanz zu enthalten, wobei der Körper (3) einen Ansatz (30) umfasst, der dazu bestimmt ist, eine Verbindungsvorrichtung aufzunehmen, und eine Öffnung (32), die in Bezug auf den Ansatz (30) auf der entgegengesetzten Seite des Körpers (3) gelegen ist,
- einen Kolben (5), der in dem Körper (3) verschieblich gelagert ist und entlang einer Längsachse (X3) der Spritze translatorisch beweglich ist zwischen einer eingeschobenen Position, in welcher der Kolben (5) auf der Seite des Ansatzes (30) am Anschlag ist, und einer ausgezogenen Position, in welcher der Kolben (5) auf der Seite der Öffnung (32) am Anschlag ist,
- Greifelemente (34, 36), die am Körper (3) vorgesehen sind, und ein Greifelement (50), das an einem Ende des Kolbens (5) vorgesehen ist, das in Bezug auf einen Kopf (57) des Kolbens (5) entgegengesetzt ist,
wobei die am Körper (3) vorgesehenen Greifelemente (34, 36) Anlagebereiche (34a, 36a) für Finger (In, M, P) eines Benutzers umfassen, die in Richtung des Greifelements (50) des Kolbens (5) ausgerichtet sind, wobei das Greifelement (50) des Kolbens (5) mindestens einen Anlagebereich (50a) für Finger (In, M, P) des Benutzers umfasst, der gegenüber den am Körper (3) vorgesehenen Greifelementen (34, 36) gelegen ist,
**dadurch gekennzeichnet, dass** der Kolben (5) aus einem einzigen Stück aus einem Material gebildet ist, das einen Elastizitätsmodul von mehr als 3000 MPa hat, und dass der Kolben (5) keinen elastischen Bereich zwischen seinem Greifelement (50) und der zu injizierenden Substanz aufweist.

2. Spritze nach Anspruch 1, **dadurch gekennzeichnet, dass** der Kolben (5) in Bezug auf den Körper (3) um die Längsachse (X3) der Spritze (1) drehbeweglich ist.

3. Spritze nach einem der vorhergehenden Ansprüche, **dadurch gekennzeichnet, dass** das Greifelement (50) des Kolbens (5) einen Anlagebereich (50b) umfasst, der entgegengesetzt zum Kopf (57) des Kolbens (5) gelegen ist.

4. Spritze nach einem der vorhergehenden Ansprüche, **dadurch gekennzeichnet, dass** der Ansatz (30) des Körpers (3) einen umfänglichen Kragen (38) umfasst, von dem eine Außenfläche mit Greifrippen (38a) versehen ist, die sich parallel zur Längsrichtung (X3) der Spritze (1) erstrecken.

5. Spritze nach einem der vorhergehenden Ansprüche, **dadurch gekennzeichnet, dass** der Anlagebereich (50a) des Greifelements (50) des Kolbens (5), der gegenüber den am Körper (3) vorgesehenen Greifelementen (34, 36) gelegen ist, konkave Formen umfasst, die beidseits der Stange (52) des Kolbens (5) vorgesehen sind.

6. Spritze nach einem der vorhergehenden Ansprüche, **dadurch gekennzeichnet, dass** das Greifelement (50) des Kolbens (5) in der eingeschobenen Position des Kolbens (5) von den am Körper (3) vorgesehenen Greifelementen (34, 36) um einen Abstand von mindestens 15 mm entfernt ist.

7. Spritze nach einem der vorhergehenden Ansprüche, **dadurch gekennzeichnet, dass** die Anlagebereiche (34a, 36a) der Greifelemente (34, 36), die am Körper (3) vorgesehen sind, konkave Formen umfassen, die beidseits der Öffnung (32) des hohlen Körpers vorgesehen sind und dem Anlagebereich des Kolbens (5) zugewandt sind, wobei diese konkaven Formen an zwei radialen Lappen ausgebildet sind, die jeweils eines der am Körper (3) vorgesehenen Greifelemente (34, 36) bilden.

8. Spritze nach einem der vorhergehenden Ansprüche, **dadurch gekennzeichnet, dass** die am Körper (3) vorgesehenen Greifelemente (34, 36) an einem einzigen Stück (37) ausgebildet sind, wobei dieses Stück (37) am Körper (3) um die Längsachse (X3) der Spritze drehbar gelagert ist.

9. Spritze nach Anspruch 8, **dadurch gekennzeichnet, dass** die beiden konkaven Anlagebereiche (40) an dem Stück (37) ausgebildet sind, an dem die am Körper (3) vorgesehenen Greifelemente (34, 36) ausgebildet sind, an den Teilen, die diese Greifelemente (34, 36) verbinden.

10. Spritze nach dem vorhergehenden Anspruch, **dadurch gekennzeichnet, dass** die beiden konkaven Anlagebereiche (40) in Richtung des Greifelements (50) ausgerichtet sind, wobei sie das Anlegen eines Fingers des Benutzers entlang einer in Bezug auf die Längsachse (X3) der Spritze radialen Richtung ermöglichen.

11. Spritze nach einem der vorhergehenden Ansprüche, **dadurch gekennzeichnet, dass** das Greifelement (50) des Kolbens (5) eine ovale Form hat.

## Claims

1. A syringe (1) comprising:
- an elongate hollow body (3) forming a reservoir intended to contain a substance to be injected, the body (3) comprising a tip (30) intended to receive a connection device, and an opening (32) located at the opposite end of the body (3) relative to the tip (30),
- a piston (5) mounted slidably in the body (3) and movable in translation along a longitudinal axis (X3) of the syringe between a retracted position, in which the piston (5) is in abutment at the tip (30), and an extended position, in which the piston (5) is in abutment at the opening (32),
- gripping members (34, 36) provided on the body (3), and a gripping member (50) provided at an opposite end of the piston (5) relative to a head (57) of the piston (5),
the gripping members (34, 36) provided on the body (3) comprising bearing zones (34a, 36a) for fingers (In, M, P) of a user, which bearing zones (34a, 36a) are oriented toward the gripping member (50) of the piston (5), the gripping member (50) of the piston (5) comprising at least one bearing zone (50a) for fingers (In, M, P) of the user, which bearing zone (50a) is located facing the gripping members (34, 36) provided on the body (3),
**characterized in that** the piston (5) is formed as a single piece made from a material having a Young's modulus greater than 3000 MPa, and **in that** the piston (5) has no elastic zone between its gripping member (50) and the substance to be injected.

2. The syringe as claimed in claim 1, **characterized in that** the piston (5) is movable in rotation with respect to the body (3) about the longitudinal axis (X3) of the syringe (1).

3. The syringe as claimed in either of the preceding claims, **characterized in that** the gripping member (50) of the piston (5) comprises a bearing zone (50b) located facing away from the head (57) of the piston (5).

4. The syringe as claimed in one of the preceding claims, **characterized in that** the tip (30) of the body (3) comprises a peripheral flange (38), an external surface of which is provided with gripping ribs (38a) extending parallel to the longitudinal axis (X3) of the syringe (1).

5. The syringe as claimed in one of the preceding claims, **characterized in that** the bearing zone (50a) of the gripping member (50) of the piston (5), located facing the gripping members (34, 36) provided on the body (3), comprises concave formations provided on each side of the rod (52) of the piston (5).

6. The syringe as claimed in one of the preceding claims, **characterized in that**, in the retracted position of the piston (5), the gripping member (50) of the piston (5) is spaced apart from the gripping members (34, 36), provided on the body (3), by a distance at least equal to 15 mm.

7. The syringe as claimed in one of the preceding claims, **characterized in that** the bearing zones (34a, 36a) of the gripping members (34, 36) provided on the body (3) comprise concave formations provided on each side of the opening (32) of the hollow body and facing the bearing zone of the piston (5), these concave formations being configured on two radial tabs, which each form one of the gripping members (34, 36) provided on the body (3).

8. The syringe as claimed in one of the preceding claims, **characterized in that** the gripping members (34, 36) provided on the body (3) are configured on one piece (37), this piece (37) being mounted rotatably on the body (3) about the longitudinal axis (X3) of the syringe.

9. The syringe as claimed in claim 8, **characterized in that** two concave bearing zones (40) are configured on the piece (37) on which the gripping members (34, 36) provided on the body (3) are configured, on the parts that connect these gripping members (34, 36).

10. The syringe as claimed in the preceding claim, **characterized in that** the two concave bearing zones (40) are oriented in the direction of the gripping member (50), allowing a finger of the user to bear in a radial direction with respect to the longitudinal axis (X3) of the syringe.

11. The syringe as claimed in one of the preceding claims, **characterized in that** the gripping member (50) of the piston (5) has an oval shape.
